# EUROPÄISCHE PATENTSCHRIFT

(11) **EP 3 093 041 B1**
(45) Veröffentlichungstag und Bekanntmachung des Hinweises auf die Patenterteilung: **25.12.2019**
(21) Anmeldenummer: 15167154.2
(22) Anmeldetag: 11.05.2015
(51) Int. Cl.: A61M 37/00

(54) **VERFAHREN ZUM AUTOMATISCHEN STEUERN EINER HAUTSTECHVORRICHTUNG, HAUTSTECHVORRICHTUNG UND STEUEREINRICHTUNG**
METHOD FOR THE AUTOMATIC CONTROL OF A SKIN PUNCTURING DEVICE, SKIN PUNCTURING DEVICE AND CONTROL DEVICE
PROCÉDÉ DE COMMANDE AUTOMATIQUE D'UN DISPOSITIF PERCUTANÉ, DISPOSITIF PERCUTANÉ ET DISPOSITIF DE COMMANDE

(43) Veröffentlichungstag der Anmeldung: 16.11.2016
(73) Patentinhaber: MT.DERM GmbH, 14167 Berlin (DE)
(72) Erfinder: Scherkowski, Dirk, 12489 Berlin (DE)
(74) Vertreter: Bittner, Thomas L.

(56) Entgegenhaltungen:
- EP-A1- 1 872 823
- WO-A2-2014/086342
- US-A1- 2011 288 575

## Beschreibung

Die Erfindung betrifft ein Verfahren zum automatischen Steuern einer Hautstechvorrichtung, eine Hautstechvorrichtung sowie eine Steuereinrichtung.

### Hintergrund

Vorrichtungen zum lokalen Aufstechen einer menschlichen oder einer tierischen Haut werden in der Regel mit einem Handstechgerät ausgeführt. Vom Bedienpersonal können derartige Handstechgeräte zum Applizieren einer Farbe für eine Tätowierung (Tattoo) und / oder permanentes Make-up im Bereich der Hautoberfläche verwendet werden. Aber auch das Applizieren kosmetischer oder medizinischer Wirkstoffe über die Haut ist mit solchen Geräten möglich, indem die Haut lokal aufgestochen wird. Darüber hinaus können solche Geräte verwendet werden, ohne dass irgendeine Substanz eingebracht wird, zum Beispiel zur Hautstimulierung.

Ein Handstechgerät zum lokalen Aufstechen einer Haut ist beispielsweise aus der Druckschrift DE 299 19 199 U1 bekannt. Das bekannte Handgerät weist ein Griffstück, eine Antriebseinrichtung und eine Stechnadel auf, die mit Hilfe der Antriebseinrichtung im Betrieb relativ zu einer Nadeldüse vor und zurück bewegt wird, wobei mindestens zwei lösbar miteinander verbundene Module vorgesehen sind, und das eine der beiden Module als wieder verwendbares Grundmodul mit integrierter Antriebseinrichtung ausgebildet ist. Das andere der beiden Module ist ein sterilisiertes Einwegmodul, in das bei dem bekannten Handgerät alle durch die Köperflüssigkeiten eines Kunden kontaminierbaren Bestandteile integriert sind. Auf diese Weise wird das Handgerät in Form von zwei Modulen zur Verfügung gestellt, von denen eines, nämlich das Einwegmodul, nach der Benutzung ausgetauscht werden kann, während das andere Modul, welches die Antriebseinrichtung umfasst, wieder verwendet wird. Mit Hilfe des Einwegmoduls werden die hygienischen Bedingungen beim Anbringen einer Tätowierung und / oder von permanentem Make-up verbessert, da alle Teile ausgetauscht werden, die potentiell durch die bei der Behandlung austretende Körperflüssigkeit des Kunden kontaminiert werden können. Es wird so vermieden, dass das gesamte Handgerät ausgetauscht werden muss.

Aus dem Dokument EP 1 495 782 A1 ist ein Antriebsmodul für ein Handstechgerät zum lokalen Aufstechen einer menschlichen oder einer tierischen Haut bekannt, bei dem eine Antriebseinrichtung, mit der eine Antriebsbewegung erzeugbar ist, und ein an die Antriebseinrichtung gekoppelter Wandlungsmechanismus vorgesehen sind, mit dem die Antriebsdrehbewegung in einer auf eine die Haut lokal aufstechende Stecheinrichtung einkoppelbare Vorwärts- / Rückwärtsbewegung umgewandelt wird, so dass eine repetierende Bewegung einer Stechnadel ermöglicht ist.

Aus dem Dokument US 2011/0288575 A1 ist eine Hautstechvorrichtung bekannt, die eingerichtet ist Steuersignale zum Betrieb über eine drahtlose Datenkommunikationsverbindung zu empfangen. Beim Betrieb der Hautstechvorrichtung wechseln sich eine Ruhebetriebsart sowie eine Arbeitsbetriebsart ab, wobei in der Arbeitsbetriebsart ein Stechwerkzeug repetierend aus- und eingefahren wird, was in der Ruhebetriebsart unterbrochen ist. Ein Übergang zwischen der Ruhebetriebsart und der Arbeitsbetriebsart findet auf den Empfang eines Einschaltbefehls statt.

### Zusammenfassung

Aufgabe der Erfindung ist es, ein Verfahren zum automatischen Steuern einer Hautstechvorrichtung und eine Hautstechvorrichtung sowie eine Steuereinrichtung anzugeben, mit denen es ermöglicht ist, verbesserte anwendungsbezogene Betriebsarten bereitzustellen.

Zur Lösung ist ein Verfahren zum automatischen Steuern einer Hautstechvorrichtung nach dem unabhängigen Anspruch 1 geschaffen. Weiterhin ist eine Hautstechvorrichtung zum lokalen Aufstechen einer menschlichen oder einer tierischen Haut nach dem unabhängigen Anspruch 9 geschaffen. Gegenstand des unabhängigen Anspruch 11 ist eine Steuereinrichtung zum Steuern eines Handstechgeräts. Ausgestaltungsvarianten sind Gegenstand von abhängigen Unteransprüchen.

Nach einem Aspekt ist ein Verfahren zum automatischen Steuern einer Hautstechvorrichtung geschaffen. Die Hautstechvorrichtung, die zum lokalen Aufstechen einer menschlichen oder einer tierischen Haut nutzbar ist, weist ein Handstechgerät mit einer Antriebseinrichtung und einer an die Antriebseinrichtung funktionell koppelnden Stecheinrichtung, die in einem Gehäuse aufgenommen sind, sowie eine Steuereinrichtung auf, die eingerichtet ist, im Betrieb Steuersignale für die Antriebseinrichtung bereitzustellen. Bei dem Verfahren wird durch die Antriebseinrichtung eine Antriebskraft bereitgestellt und auf ein Stechwerkzeug der Stecheinrichtung eingekoppelt, derart, dass das Stechwerkzeug beim Ausführen einer Arbeitsbewegung aus- und eingefahren wird. Die Arbeitsbewegung wird bewirkt durch die auf das Stechwerkzeug eingekoppelte Antriebskraft, wobei hierbei das Stechwerkzeug vor- und zurückbewegt wird. Bei dem Verfahren wird eine Benutzereingabe für einen Intervallbetrieb des Handstechgeräts erfasst. Bei dem Intervallbetrieb wiederholen sich abwechselnd Arbeitsintervalle, in denen aufgrund des Betriebs der Antriebseinrichtung das Stechwerkzeug mit einer Wiederholfrequenz mehrere Arbeitsbewegungen ausführt, und Nichtarbeitsintervalle, in denen der Betrieb der Antriebseinrichtung unterbrochen ist. Die Antriebskraft wird dann nicht bereitgestellt, so dass das Stechwerkzeug keine Arbeitsbewegung ausführt. Es werden Steuersignale für die Antriebseinrichtung gemäß dem Intervallbetrieb durch die Steuereinrichtung erzeugt und an die Antriebseinrichtung übertragen, welche den Steuersignalen entsprechend betrieben wird.

Nach einem weiteren Aspekt ist eine Hautstechvorrichtung zum lokalen Aufstechen einer menschlichen oder einer tierischen Haut geschaffen. Die Hautstechvorrichtung weist ein Handstechgerät mit einer Antriebseinrichtung und einer an die Antriebseinrichtung funktionell koppelnden Stecheinrichtung auf, die in einem Gehäuse aufgenommen sind, sei es in einem gemeinsamen Gehäuse oder getrennten Gehäusen. Die Antriebseinrichtung ist eingerichtet, eine Antriebskraft bereitzustellen und auf ein Stechwerkzeug der Stecheinrichtung einzukoppeln, derart, dass das Stechwerkzeug beim Ausführen einer Arbeitsbewegung aus- und eingefahren wird. Die Hautstechvorrichtung verfügt über eine Steuereinrichtung, die eingerichtet ist, einen Intervallbetrieb des Handstechgeräts zu steuern. Beim Intervallbetrieb wird eine Benutzereingabe für den Intervallbetrieb erfasst, bei dem sich Arbeitsintervalle, in denen aufgrund des Betriebs der Antriebseinrichtung das Stechwerkzeug mit einer Wiederholfrequenz mehrere Arbeitsbewegungen ausführt, und Nichtarbeitsintervalle, in denen der Betrieb der Antriebseinrichtung unterbrochen ist, mit einem vorgegebenen zeitlichen Abstand abwechselnd wiederholt. Die Steuereinrichtung ist eingerichtet, zum Intervallbetrieb Steuersignale für die Antriebseinrichtung gemäß dem Intervallbetrieb zu erzeugen und an die Antriebseinrichtung zu übertragen, so dass die Antriebseinrichtung den Steuersignalen entsprechend betreibbar ist.

Ein weiterer Aspekt bezieht sich auf eine Steuereinrichtung zum Steuern eines Handstechgeräts zum lokalen Aufstechen einer Haut mit einer Datenschnittstelle, die eingerichtet ist, Steuersignale für einen Intervallbetrieb eines Handstechgeräts auszugeben, wenn das Handstechgerät an die Datenschnittstelle gekoppelt ist, wobei die Steuereinrichtung eingerichtet ist, eine Benutzereingabe für den Intervallbetrieb zu erfassen, bei dem sich Arbeitsintervalle, in denen aufgrund des Betriebs einer Antriebseinrichtung ein Stechwerkzeug des Handstechgeräts mit einer Wiederholfrequenz mehrere Arbeitsbewegungen ausführt, und Nichtarbeitsintervalle, in denen der Betrieb der Antriebseinrichtung unterbrochen ist, mit einem vorgegebenen zeitlichen Abstand abwechselnd wiederholen, und Steuersignale für die Antriebseinrichtung gemäß dem Intervallbetrieb zu erzeugen und an der Datenschnittstelle bereitzustellen, so dass die Antriebseinrichtung den Steuersignalen entsprechend betreibbar ist. Das Handstechgerät kann über einer drahtlose oder eine kabelgebundene Verbindung an die Datenschnittstelle koppeln.

Die automatische Steuerung der Hautstechvorrichtung gemäß dem Intervallbetrieb ermöglicht es dem Nutzer insbesondere, während der Nichtarbeitsintervalle das Handstechgerät zwischen einer Applikationsstelle auf der aufzustechenden Haut und einer weiteren Applikationsstelle zu verlagern, ohne dass der Nutzer hierzu aktiv in die Steuerung der Hautstechvorrichtung eingreifen muss, beispielsweise mit einem Fußschalter. Im Fall eines Handstechgeräts mit Abgabe einer Flüssigkeit kann unkontrolliertes Abgeben vermieden werden. Auch kann erreicht werden, dass bereits die erste Ausfahrbewegung des Stechwerkzeugs auf die gewünschte Stechtiefe in der Haut erfolgen kann.

Im Vergleich zu bekannten Hautstechvorrichtungen ist die Betriebsartenauswahl für den Nutzer erweitert, insbesondere in Ergänzung zu der üblichen Maßnahme des Veränderns der Wiederholfrequenz für die Arbeitsbewegung.

Hautstechvorrichtungen mit einem Handstechgerät sind als solche in verschiedenen Ausführungsformen bekannt. Sie werden benutzt, um eine menschliche oder eine tierische Haut lokal aufzustechen, sei es zum Beispiel zum Einbringen eines Farbstoffs oder eines medizinischen oder kosmetischen Wirkstoffes oder zur Hautstimulierung ohne Applikation einer Substanz. Die hier vorgeschlagenen neuen Technologien können bei einer oder mehreren der bekannten Nutzungsarten zum Einsatz kommen.

Der Intervallbetrieb kann mittels Benutzerbetätigung gestartet und / oder beendet werden, zum Beispiel mithilfe des Betätigens eines Fußschalters, der an die Steuereinrichtung koppelt.

Die Hautstechvorrichtung kann als eine Vorrichtung zum Eintragen einer Substanz in die Haut oder als eine Vorrichtung zur Hautstimulierung, insbesondere zum Stimulieren von Haarfollikel, ausgeführt sein. Die Vorrichtung zum Substanzeintrag kann zum Beispiel ein Tattoogerät sein.

Während des Arbeitsintervalls können mehrere Arbeitsbewegungen mit einer Wiederholfrequenz ausgeführt werden. Die Arbeitsbewegung kann als Hubbewegung (Vor- und Zurückbewegen) ausgeführt werden. Bei dieser Ausführungsform ist eine Mehrfachhubbewegung vorgesehen, die sich durch die Wiederholung der Arbeitsbewegungen von der Einzelhubbewegung unterscheidet.

Die mehreren Arbeitsbewegungen können während des Arbeitsintervalls mit einer Wiederholfrequenz von wenigstens 50 Hz ausgeführt werden. Die Wiederholfrequenz kann zwischen etwa 50 Hz und 200 Hz betragen.

Eine zeitliche Intervalllänge des Nichtarbeitsintervalls kann größer sein als der zeitliche Abstand zwischen aufeinanderfolgenden Arbeitsbewegungen im Arbeitsintervall. Die zeitliche Intervalllänge des Nichtarbeitsintervalls, also der Zeitraum zwischen dem Beginn und dem Ende eines Nichtarbeitsintervalls, kann um ein Vielfaches größer sein, als der zeitliche Abstand zwischen aufeinanderfolgenden Arbeitsbewegungen innerhalb des Arbeitsintervalls. Letzteres entspricht der Periodendauer der jeweils genutzten Wiederholfrequenz für die Arbeitsbewegungen.

Die zeitliche Intervalllänge des Nichtarbeitsintervalls kann größer sein als eine zeitliche Intervalllänge des Arbeitsintervalls.

Die Steuereinrichtung kann in einem Steuergerät angeordnet sein, welches getrennt von dem Handgerät gebildet und mit diesem verbunden ist, derart, dass wenigstens die erzeugten Steuersignale von dem Steuergerät an das Handstechgerät übertragbar sind, wobei die Benutzereingabe über eine Eingabeeinrichtung des Steuergeräts erfasst werden. Alternativ kann die Steuereinrichtung in oder an dem Gehäuse des Handstechgeräts aufgenommen sein. An das gemeinsame Gehäuse kann insbesondere eine Energieversorgungsleitung angeschlossen, sei es zeitweise oder dauerhaft. Die Steuersignalübertragung zwischen dem Steuergerät und dem Handstechgerät kann über eine kabellose und / oder eine kabelgebundene Datenübertragungsverbindung erfolgen.

Mit dem Erfassen der Benutzereingabe kann eine Auswahl aus mehreren vorgegebenen Intervallbetriebsarten erfasst werden. Hierbei werden dem Benutzer mehrere vorbestimmte Intervallbetriebsarten zur Auswahl angeboten, beispielsweise über ein Display einer Anzeigeeinrichtung, die an dem Steuergerät vorgesehen sein kann. Die mehreren Intervallbetriebsarten können sich hinsichtlich eines oder mehrerer den Intervallbetrieb charakterisierender Parameter unterscheiden. Es kann alternativ vorgesehen sein, dass dem Benutzer eine Auswahl verschiedener Behandlungsmethoden zur Verfügung gestellt wird, um die Benutzerauswahl einer Behandlungsmethode zu erfassen. In dem Steuergerät, beispielweise einem elektronischen Datenspeicher, ist dann für die jeweilige Behandlungsmethode eine Intervallbetriebsart hinterlegt, die als Reaktion auf die Benutzerauswahl betreffend die Behandlungsmethode ausgewählt wird, um mit Hilfe der Steuereinrichtung entsprechende Steuersignale zu erzeugen.

Mit dem Erfassen der Benutzereingabe kann eine Benutzervorgabe für wenigstens einen Intervallbetriebsparameter ausgewählt aus der folgenden Gruppe von Intervallbetriebsparametern erfasst werden: zeitliche Intervalllänge des Arbeitsintervalls, zeitliche Intervalllänge des Nichtarbeitsintervalls, Anzahl von Wiederholungen der Arbeitsbewegung pro Arbeitsintervall, Wiederholfrequenz der Arbeitsbewegungen im Arbeitsintervall und zeitliche Länge des Intervallbetriebs. Es kann vorgesehen sein, dass als Reaktion auf das Erfassen der einen oder der mehreren Benutzervorgaben eine individuelle Intervallbetriebsart in der Steuereinrichtung erzeugt wird, die verschieden ist von eventuell bereitgestellten vorgegebenen Intervallbetriebsarten. Das Erfassen dieser oder anderer Benutzereingaben kann über unterschiedliche Eingabeeinrichtungen erfolgen, zum Beispiel einen berührungssensitiven Bildschirm und / oder Tastmittel.

Die Steuereinrichtung kann in einem Steuergerät angeordnet sein, welches getrennt von dem Handgerät gebildet und mit diesem verbunden ist, derart, dass wenigstens die erzeugten Steuersignale von dem Steuergerät an das Handgerät übertragbar sind.

In Verbindung mit der Hautstechvorrichtung zum lokalen Aufstechen einer menschlichen oder einer tierischen Haut können die vorangehend im Zusammenhang mit dem Verfahren zum automatischen Steuern der Handstechvorrichtung angegebenen Ausgestaltungen entsprechend vorgesehen sein.

### Beschreibung von Ausführungsbeispielen

Nachfolgend werden Ausführungsbeispiele unter Bezugnahme auf Figuren einer Zeichnung näher erläutert. Hierbei zeigen:
- Fig. 1: eine schematische perspektivische Darstellung eines Handstechgeräts mit einem Stechwerkzeug in einer eingefahren Stellung,
- Fig. 2: eine schematische perspektivische Darstellung des Handstechgeräts aus Fig. 1 mit dem Stechwerkzeug in einer ausgefahrenen Stellung und
- Fig. 3: eine schematische Darstellung einer Hautstechvorrichtung mit einem Handstechgerät und einem Steuergerät.

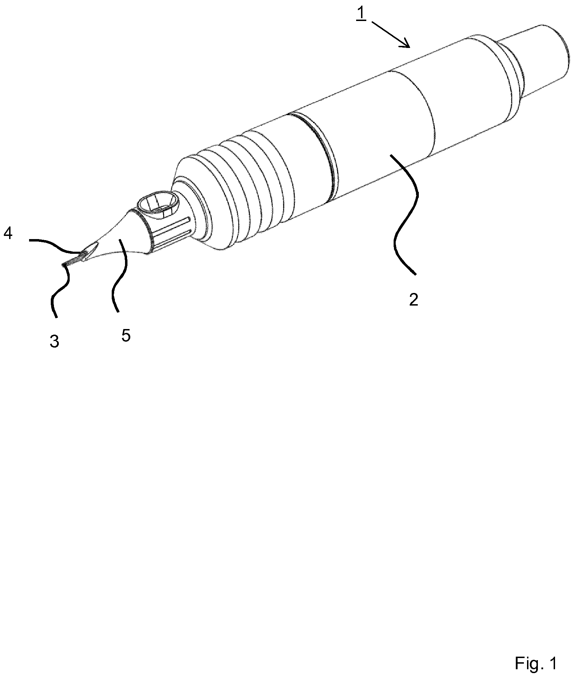

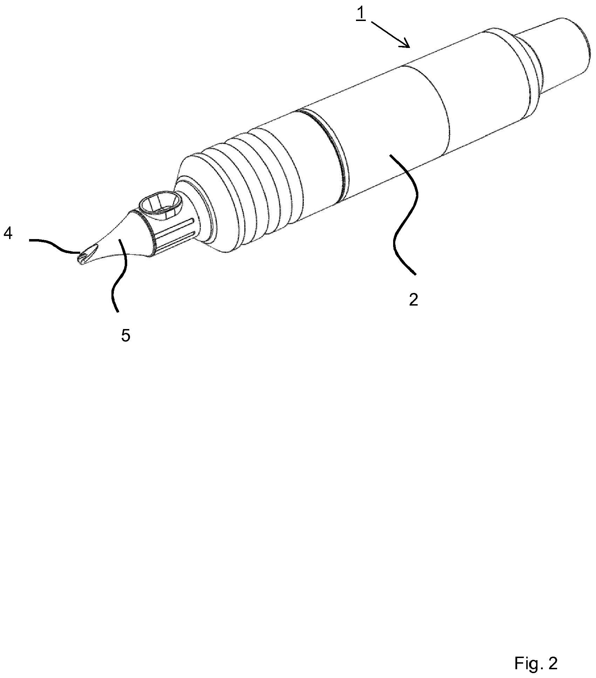

Fig. 1 und 2 zeigen schematische Darstellungen eines Hautstechgeräts 1, welches modulartig aufgebaut ist. In einem Gehäuse 2 sind eine Antriebseinrichtung sowie ein Kopplungsmechanismus (nicht dargestellt) aufgenommen, mit dem eine von der Antriebseinrichtung erzeugte Antriebskraft auf eine Stecheinrichtung gekoppelt wird, die in der dargestellten Ausführungsform als Nadelgruppe 3 mit mehreren Stechnadeln gebildet ist. Die Nadelgruppe 3 ist in den Fig.1 und 2 in einer ausgefahrenen und einer eingefahrenen Stellung gezeigt. In der ausgefahrenen Stellung steht die Nadelgruppe 3 über einer Nadelöffnung 4 an einem Nadelmodul 5 hinaus ab. Das Hautstechgerät 1 kann mit einem Steuergerät kombiniert werden, was nachfolgend näher erläutert wird.

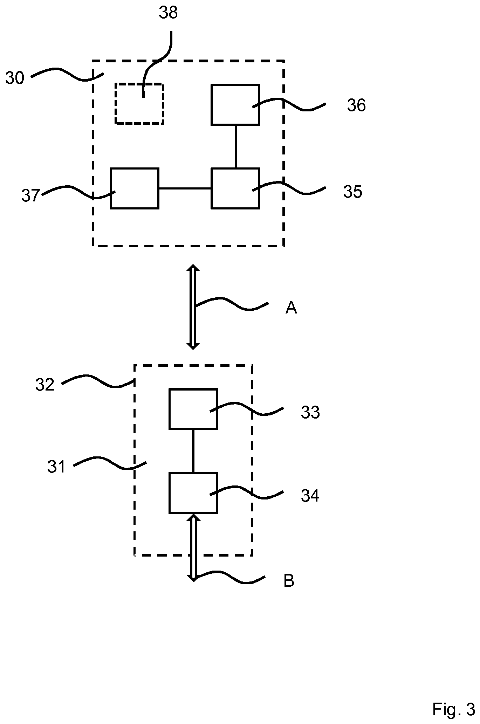

Fig. 3 zeigt eine schematische Darstellung von Komponenten einer Hautstechvorrichtung, die in der gezeigten Ausführungsform ein Steuergerät 30 und ein Handstechgerät 31 aufweist. Das Steuergerät 30 ist bei der dargestellten Ausgestaltung getrennt von dem Handstechgerät 31 ausgebildet. Zwischen dem Steuergerät 30 und dem Handstechgerät 31 sind unidirektional oder bidirektional elektronische Signale austauschbar, sei es über eine kabellose oder eine kabelgebundene Verbindung, was in Fig. 3 mittels eines Pfeils A schematisch dargestellt ist.

Mit Hilfe des Steuergeräts 30 werden insbesondere Steuersignale erzeugt, die zum Betrieb des Handstechgeräts 31 an dieses übertragen werden. Das Handstechgerät 31 verfügt in einem Gehäuse 32 über eine Antriebseinrichtung 33 und eine hieran funktionell koppelnde Stecheinrichtung 34. Mit der Antriebseinrichtung 33 wird eine Antriebskraft bereitgestellt, beispielsweise mit Hilfe eines Elektromotors, die auf die Stecheinrichtung 34 eingekoppelt wird, um ein Stechwerkzeug aus- und einzufahren, was in Fig. 3 schematisch mit Hilfe eines Pfeils B gezeigt ist. Das Stechwerkzeug kann eine oder mehrere Stechnadeln aufweisen, die geeignet sind, eine menschliche oder eine tierische Haut lokal aufzustechen.

Die für den Betrieb der Antriebseinrichtung 33 vorgesehenen Steuersignale werden mittels des Steuergeräts 30 erzeugt. Dieses verfügt über eine Steuereinrichtung 35, die in der gezeigten Ausführungsform an eine Eingabeeinrichtung 36 sowie an eine Anzeigevorrichtung 37 koppelt. Mit Hilfe der Eingabeeinrichtung 36 werden Benutzereingaben erfasst. Die Eingabeeinrichtung 36 kann zum Beispiel eine Tastatur umfassen. Auch ein sensitiver Touchscreen kann vorgesehen sein, der dann zusammen mit der Anzeigeeinrichtung 37 ausgebildet ist. Sowohl das Handstechgerät 31 wie auch das Steuergerät 30 können weitere Funktionskomponenten oder -baugruppen umfassen, was in Fig. 3 mittels der Funktionskomponente 38 schematisch gezeigt ist.

Mit Hilfe der Steuereinrichtung 35 können am Steuergerät 30 vorgegebene Intervallbetriebsarten bereitgestellt werden, zwischen denen der Benutzer mittels Eingabe über die Eingabeeinrichtung 36 auswählen kann. Darüber hinaus kann der Benutzer über die Eingabevorrichtung 36 individuelle Betriebsparameter eingeben. Die Steuereinrichtung 35 erzeugt Steuersignale für die Intervallbetriebsart, bei der sich Arbeitsintervalle und Nichtarbeitsintervalle einem vom Benutzer gewählten Betriebsmodus entsprechend abwechseln.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Verfahren zum automatischen Steuern einer Hautstechvorrichtung, wobei die Hautstechvorrichtung ein Handstechgerät mit einer Antriebseinrichtung und einer an die Antriebseinrichtung funktionell koppelnden Stecheinrichtung, die in einem Gehäuse aufgenommen sind, sowie eine Steuereinrichtung aufweist, die eingerichtet ist, im Betrieb Steuersignale für die Antriebseinrichtung bereitzustellen, und wobei das Verfahren die folgenden Schritte aufweist:
- Bereitstellen einer Antriebskraft durch die Antriebseinrichtung und Koppeln der Antriebskraft auf ein Stechwerkzeug der Stecheinrichtung, derart, dass das Stechwerkzeug beim Ausführen einer Arbeitsbewegung aus- und eingefahren wird,
- Erfassen einer Benutzereingabe für einen Intervallbetrieb des Handstechgeräts, bei dem sich Arbeitsintervalle, in denen aufgrund des Betriebs der Antriebseinrichtung das Stechwerkzeug mit einer Wiederholfrequenz mehrere Arbeitsbewegungen ausführt, und Nichtarbeitsintervalle, in denen der Betrieb der Antriebseinrichtung unterbrochen ist, mit einem vorgegebenen zeitlichen Abstand abwechselnd wiederholen,
- Erzeugen von Steuersignalen für die Antriebseinrichtung gemäß dem Intervallbetrieb durch die Steuereinrichtung und Übertragen der Steuersignale an die Antriebseinrichtung und
- Betreiben der Antriebseinrichtung den Steuersignalen entsprechend.

2. Verfahren nach Anspruch 1, **dadurch gekennzeichnet, dass** während des Arbeitsintervalls mehrere Arbeitsbewegungen mit einer Wiederholfrequenz ausgeführt werden.

3. Verfahren nach Anspruch 2, **dadurch gekennzeichnet, dass** die mehreren Arbeitsbewegungen während des Arbeitsintervalls mit einer Wiederholfrequenz von wenigstens 1Hz ausgeführt werden.

4. Verfahren nach mindestens einem der vorangehenden Ansprüche, **dadurch gekennzeichnet, dass** eine zeitliche Länge des Nichtarbeitsintervalls größer ist als der zeitliche Abstand zwischen aufeinanderfolgenden Arbeitsbewegungen im Arbeitsintervall.

5. Verfahren nach mindestens einem der vorangehenden Ansprüche, **dadurch gekennzeichnet, dass** die zeitliche Länge des Nichtarbeitsintervalls größer ist als eine zeitliche Intervalllänge des Arbeitsintervalls.

6. Verfahren nach mindestens einem der vorangehenden Ansprüche, **dadurch gekennzeichnet, dass** die Steuereinrichtung in einem Steuergerät angeordnet ist, welches getrennt von dem Handstechgerät gebildet und mit diesem verbunden ist, derart, dass wenigstens die erzeugten Steuersignale von dem Steuergerät an das Handstechgerät übertragbar sind, wobei die Benutzereingabe über eine Eingabeeinrichtung des Steuergeräts erfasst wird.

7. Verfahren nach mindestens einem der vorangehenden Ansprüche, **dadurch gekennzeichnet, dass** mit dem Erfassen der Benutzereingabe eine Auswahl aus mehreren vorgegebenen Intervallbetriebsarten erfasst wird.

8. Verfahren nach mindestens einem der vorangehenden Ansprüche, **dadurch gekennzeichnet, dass** mit dem Erfassen der Benutzereingabe eine Benutzervorgabe für wenigstens einen Intervallbetriebsparameter ausgewählt aus der folgenden Gruppe von Intervallbetriebsparametern erfasst wird: zeitliche Intervalllänge des Arbeitsintervalls, zeitliche Intervalllänge des Nichtarbeitsintervalls, Anzahl von Wiederholungen der Arbeitsbewegung pro Arbeitsintervall, Wiederholfrequenz der Arbeitsbewegungen im Arbeitsintervall und zeitliche Länge des Intervallbetriebs.

9. Hautstechvorrichtung zum lokalen Aufstechen einer menschlichen oder einer tierischen Haut, mit
- einem Handstechgerät mit einer Antriebseinrichtung und einer an die Antriebseinrichtung funktionell koppelnden Stecheinrichtung, die in einem Gehäuse aufgenommen sind, wobei die Antriebseinrichtung eingerichtet ist, eine Antriebskraft bereitzustellen und auf ein Stechwerkzeug der Stecheinrichtung einzukoppeln, derart, dass das Stechwerkzeug beim Ausführen einer Arbeitsbewegung aus- und eingefahren wird, und
- einer Steuereinrichtung, die eingerichtet ist, einen Betrieb des Handstechgeräts zu steuern, indem Steuersignale für die Antriebseinrichtung erzeugt und an diese übertragen werden, so dass die Antriebseinrichtung den Steuersignalen entsprechend betrieben wird,
**dadurch gekennzeichnet, dass** die Steuereinrichtung eingerichtet ist, einen Intervallbetrieb des Handstechgeräts zu steuern und hierbei:
- eine Benutzereingabe für den Intervallbetrieb zu erfassen, bei dem sich Arbeitsintervalle, in denen aufgrund des Betriebs der Antriebseinrichtung das Stechwerkzeug mit einer Wiederholfrequenz mehrere Arbeitsbewegungen ausführt, und Nichtarbeitsintervalle, in denen der Betrieb der Antriebseinrichtung unterbrochen ist, mit einem vorgegebenen zeitlichen Abstand abwechselnd wiederholen, und
- Steuersignale für die Antriebseinrichtung gemäß dem Intervallbetrieb zu erzeugen und an die Antriebseinrichtung zu übertragen, so dass die Antriebseinrichtung den Steuersignalen entsprechend im Intervallbetrieb betrieben wird.

10. Hautstechvorrichtung nach Anspruch 9, **dadurch gekennzeichnet, dass** die Steuereinrichtung in einem Steuergerät angeordnet ist, welches getrennt von dem Handstechgerät gebildet und mit diesem verbunden ist, derart, dass wenigstens die erzeugten Steuersignale von dem Steuergerät an das Handstechgerät übertragbar sind.

11. Steuereinrichtung zum Steuern eines Handstechgeräts zum lokalen Aufstechen einer Haut, mit einer Datenschnittstelle, die eingerichtet ist, Steuersignale für einen Intervallbetrieb eines Handstechgeräts auszugeben, wenn das Handstechgerät an die Datenschnittstelle gekoppelt ist, wobei die Steuereinrichtung eingerichtet ist, eine Benutzereingabe zu erfassen und Steuersignale für die Antriebseinrichtung gemäß der Benutzereingabe zu erzeugen und an der Datenschnittstelle bereitzustellen, so dass die Antriebseinrichtung den Steuersignalen entsprechend betreibbar ist,
**dadurch gekennzeichnet, dass** die Steuereinrichtung eingerichtet ist,
- eine Benutzereingabe für den Intervallbetrieb zu erfassen, bei dem sich Arbeitsintervalle, in denen aufgrund des Betriebs einer Antriebseinrichtung ein Stechwerkzeug des Handstechgeräts mit einer Wiederholfrequenz mehrere Arbeitsbewegungen ausführt, und Nichtarbeitsintervalle, in denen der Betrieb der Antriebseinrichtung unterbrochen ist, mit einem vorgegebenen zeitlichen Abstand abwechselnd wiederholen, und
- Steuersignale für die Antriebseinrichtung gemäß dem Intervallbetrieb zu erzeugen und an der Datenschnittstelle bereitzustellen, so dass die Antriebseinrichtung den Steuersignalen entsprechend im Intervallbetrieb betreibbar ist.

## Claims

1. A method for automatically controlling a skin piercing system, wherein the skin piercing system has a manual piercing device with a driving attachment, and a piercing unit that functionally couples with the driving attachment, which are accommodated in a housing, as well as a controller, which is set up to provide control signals for the driving attachment during operation, and wherein the method comprises the following steps:
- providing a driving force with the driving attachment and coupling the driving force to a piercing tool of the piercing unit, such that the piercing tool is moved in and out while executing a working motion,
- acquiring a user input for an interval operation of the manual piercing device, during which working intervals, in which the piercing tool executes several working motions with a repetition frequency owing to the operation of the driving attachment, and non-working intervals, in which the operation of the driving attachment is interrupted, are alternatingly repeated with a prescribed time lag,
- generating control signals for the driving attachment based on the interval operation with the controller and transmitting the control signals to the driving attachment; and
- operating the driving attachment according to the control signals.

2. The method according to claim 1, **characterized in that** several working motions are executed with a repetition frequency during the working interval.

3. The method according to claim 2, **characterized in that** the several working motions during the working interval are executed with a repetition frequency of at least 1 Hz.

4. The method according to at least one of the preceding claims, **characterized in that** a duration of the non-working interval is greater than the time lag between sequential working motions in the working interval.

5. The method according to at least one of the preceding claims, **characterized in that** the duration of the non-working interval is greater than an interval duration of the working interval.

6. The method according to at least one of the preceding claims, **characterized in that** the controller is arranged in a control device, which is separate from the manual piercing device and connected with the latter, such that at least the generated control signals can be transmitted from the control device to the manual piercing device, wherein the user input is acquired via an input unit of the control device.

7. The method according to at least one of the preceding claims, **characterized in that** a selection of several prescribed interval operation types is acquired with the acquisition of the user input.

8. The method according to at least one of the preceding claims, **characterized in that** a user default for at least one interval operation parameter selected from the following group of interval operation parameters is acquired with the acquisition of the user input:
interval duration of the working interval, interval duration of the non-working interval,
number of repetitions of the working motion per working interval, repetition frequency of the working motions in the working interval, and duration of interval operation.

9. A skin piercing device for locally piercing human or animal skin, with
- a manual piercing device with a driving attachment and a piercing unit that functionally couples with the driving attachment, which are accommodated in a housing, wherein the driving attachment is designed to provide a driving force and couple it to a piercing tool of the piercing unit, such that the piercing tool is moved in and out while executing a working motion,
- a controller designed to control the operation of the manual piercing device by generating control signals for the driving attachment and transmitting them to the latter, so that the driving attachment is operated according to the control signals,
**characterized in that** the controller is designed to control an interval operation of the manual piercing device, and in so doing:
- acquire a user input for the interval operation, during which working intervals, in which the piercing tool executes several working motions with a repetition frequency owing to the operation of the driving attachment, and non-working intervals, in which the operation of the driving attachment is interrupted, are alternatingly repeated with a prescribed time lag, and
- generate control signals for the driving attachment based on the interval operation and transmit them to the driving attachment, so that the driving attachment is operated in the interval mode according to the control signals.

10. The skin piercing device according to claim 9, **characterized in that** the controller is arranged in a control device, which is separate from the manual piercing device and connected with the latter, such that at least the generated control signals can be transmitted from the control device to the manual piercing device.

11. A controller for controlling a manual piercing device for locally piercing the skin, with a data interface designed to output control signals for an interval operation if the manual piercing device is coupled to the data interface, wherein the controller is designed to acquire a user input and generate control signals for the driving attachment according to the user input and provide the latter at the data interface, so that the driving attachment can be operated according to the control signals,
**characterized in that** the controller is designed to:
- acquire a user input for the interval operation, during which working intervals, in which a piercing tool of the manual piercing device executes several working motions with a repetition frequency owing to the operation of a driving attachment, and non-working intervals, in which the operation of the driving attachment is interrupted, are alternatingly repeated with a prescribed time lag, and
- generate control signals for the driving attachment based on the interval operation and provide them at the data interface, so that the driving attachment can be operated in the interval mode according to the control signals.

## Revendications

1. Procédé pour la commande automatique d'un dispositif de piquage de peau, dans lequel le dispositif de piquage de peau présente un appareil piqueur manuel avec un dispositif d'entrainement et un dispositif piqueur couplé de manière fonctionnelle au dispositif d'entraînement, lesquels sont logés dans un boîtier, ainsi qu'un dispositif de commande, lequel est étudié, lors du fonctionnement, pour mettre à disposition des signaux de commande pour le dispositif d'entraînement, et dans lequel le procédé présente les étapes suivantes :
- la mise à disposition d'une force d'entraînement par le dispositif d'entraînement et l'application de la force d'entraînement à un outil de piquage du dispositif piqueur de telle sorte que l'outil de piquage est sorti et rentré lors de l'exécution d'un mouvement de travail,
- la saisie d'une entrée d'utilisateur pour un fonctionnement intermittent de l'appareil piqueur manuel où des intervalles de travail au cours desquels, du fait du fonctionnement du dispositif d'entraînement, l'outil de piquage exécute plusieurs mouvements de travail avec une fréquence de répétition, et des intervalles de non-travail au cours desquels le fonctionnement du dispositif d'entraînement est interrompu, se répètent en alternance selon un intervalle de temps prescrit,
- la génération de signaux de commande pour le dispositif d'entraînement conformément au fonctionnement intermittent par le dispositif de commande, et la transmission des signaux de commande au dispositif d'entraînement, et
- l'exploitation du dispositif d'entraînement conformément aux signaux de commande.

2. Procédé selon la revendication 1, **caractérisé en ce que**, pendant l'intervalle de travail, plusieurs mouvements de travail sont réalisés avec une fréquence de répétition.

3. Procédé selon la revendication 2, **caractérisé en ce que** les plusieurs mouvements de travail pendant l'intervalle de travail sont exécutés avec une fréquence de répétition d'au moins un 1Hz.

4. Procédé selon l'une au moins des revendications précédentes, **caractérisé en ce qu'**une longueur de temps de l'intervalle de non-travail est supérieure à l'intervalle de temps entre des mouvements de travail successifs dans l'intervalle de travail.

5. Procédé selon l'une au moins des revendications précédentes, **caractérisé en ce que** la longueur de temps de l'intervalle de non-travail est supérieure à une longueur d'intervalle de temps de l'intervalle de travail.

6. Procédé selon l'une au moins des revendications précédentes, **caractérisé en ce que** le dispositif de commande est disposé dans un appareil de commande qui est formé de manière séparée de l'appareil piqueur manuel et est relié à celui-ci, de telle sorte qu'au moins les signaux de commande générés par l'appareil de commande peuvent être transmis à l'appareil piqueur manuel, dans lequel l'entrée d'utilisateur est saisie par l'intermédiaire d'un dispositif de saisie de l'appareil de commande.

7. Procédé selon l'une au moins des revendications précédentes, **caractérisé en ce qu'**avec la saisie de l'entrée d'utilisateur, on saisit une sélection de plusieurs types de fonctionnement intermittents prescrits.

8. Procédé selon l'une au moins des revendications précédentes, **caractérisé en ce qu'**avec la saisie de l'entrée d'utilisateur, on saisit une préférence utilisateur pour au moins un paramètre de fonctionnement intermittent sélectionné dans le groupe suivant de paramètres de fonctionnement intermittent : longueur d'intervalle de temps de l'intervalle de travail, longueur d'intervalle de temps de l'intervalle de non-travail, nombre de répétitions du mouvement de travail par intervalle de travail, fréquence de répétition des mouvements de travail dans l'intervalle de travail et longueur de temps du fonctionnement intermittent.

9. Dispositif de piquage de peau pour le perçage local d'une peau humaine ou animale, avec
- un appareil piqueur manuel avec un dispositif d'entraînement et un dispositif piqueur couplé de manière fonctionnelle au dispositif d'entraînement, lesquels sont logés dans un boîtier, dans lequel le dispositif d'entraînement est étudié pour mettre à disposition une force d'entraînement et l'appliquer à un outil de piquage du dispositif piqueur de telle sorte que l'outil de piquage est rentré et sorti lors de l'exécution d'un mouvement de travail, et
- un dispositif de commande qui est étudié pour commander un fonctionnement de l'appareil piqueur manuel en ce que des signaux de commande sont générés pour le dispositif d'entraînement et sont transmis à celui-ci, de sorte que le dispositif d'entraînement est exploité conformément aux signaux de commande,
**caractérisé en ce que** le dispositif de commande est étudié pour commander un fonctionnement intermittent de l'appareil piqueur manuel et ce faisant :
- saisir une entrée d'utilisateur pour le fonctionnement intermittent où des intervalles de travail au cours desquels, en raison du fonctionnement du dispositif d'entraînement, l'outil de piquage exécute plusieurs mouvements de travail avec une fréquence de répétition, et des intervalles de non-travail au cours desquels le fonctionnement du dispositif d'entraînement est interrompu, se répètent en alternance avec un intervalle de temps prescrit, et
- générer des signaux de commande pour le dispositif d'entraînement conformément au fonctionnement intermittent et les transmettre au dispositif d'entraînement de sorte que le dispositif d'entraînement est exploité en fonctionnement intermittent conformément aux signaux de commande.

10. Dispositif de piquage de peau selon la revendication 9, **caractérisé en ce que** le dispositif de commande est disposé dans un appareil de commande qui est formé séparément de l'appareil piqueur manuel et est relié à celui-ci, de telle sorte qu'au moins les signaux de commande générés peuvent être transmis de l'appareil de commande à l'appareil piqueur manuel.

11. Dispositif de commande pour commander un appareil piqueur manuel pour le perçage local d'une peau, avec une interface de données qui est étudiée pour faire sortir des signaux de commande pour un fonctionnement intermittent d'un appareil piqueur manuel lorsque l'appareil piqueur manuel est couplé avec l'interface de données, dans lequel le dispositif de commande est étudié pour saisir une entrée d'utilisateur et générer des signaux de commande pour le dispositif d'entraînement conformément à l'entrée d'utilisateur et les mettre à disposition à l'interface de données de sorte que le dispositif d'entraînement peut être exploité conformément aux signaux de commande, **caractérisé en ce que** le dispositif de commande est étudié pour :
- saisir une entrée d'utilisateur pour le fonctionnement intermittent où des intervalles de travail au cours desquels, en raison du fonctionnement d'un dispositif d'entraînement, un outil de piquage de l'appareil piqueur manuel exécute plusieurs mouvements de travail avec une fréquence de répétition, et des intervalles de non-travail au cours desquels le fonctionnement du dispositif d'entraînement est interrompu, se répètent en alternance avec un intervalle de temps prescrit, et
- générer des signaux de commande pour le dispositif d'entraînement conformément au fonctionnement intermittent et les mettre à disposition au niveau de l'interface de données de sorte que le dispositif d'entraînement peut être exploité selon le fonctionnement intermittent conformément aux signaux de commande.
